# EUROPEAN PATENT SPECIFICATION

(11) **EP 4 099 948 B1**
(45) Date of publication and mention of the grant of the patent: **22.07.2026**
(21) Application number: 21703859.5
(22) Date of filing: 01.02.2021
(51) Int. Cl.: A61F 2/16, G02B 5/18, G02B 27/00, G02C 7/04

(54) **MULTIFOCAL OPHTHALMIC INTRAOCULAR LENS**
MULTIFOKALE OPHTHALMISCHE INTRAOKULARLINSE
LENTILLE INTRAOCULAIRE OPHTALMIQUE MULTIFOCALE

(30) Priority: 03.02.2020 DE 102020102650
(43) Date of publication of application: 14.12.2022
(73) Proprietor: Carl Zeiss Meditec AG, 07745 Jena (DE)
(72) Inventor: FORBES, Greg, Sydney, New South Wales NSW2121 (AU); KIRCHNER, Friedrich, 10589 Berlin (DE); FLESCH, Andreas, 71696 Moeglingen (DE)
(74) Representative: Patentanwälte Geyer, Fehners & Partner mbB
(86) International application number: PCT/EP2021/052302
(87) International publication number: WO 2021/156203

(56) References cited:
- WO-A2-2009/027438
- AU-A1- 2017 225 592
- AU-A1- 2017 369 976
- US-A1- 2008 300 679
- US-A1- 2019 365 528

## Description

The present invention relates to a multifocal ophthalmic intraocular lens (IOL) to be inserted into the human eye, e.g. as replacement lens in cataract surgery or in case of aphakia or as an add-on lens. The intraocular lens comprises a lens body having a surface and at least two concentric zones, with a first central zone having a first diffractive structure comprising widths and heights, and a second ring-shaped zone having a second diffractive structure comprising widths and heights.

Such a lens is known from US 2010/0312337 A1. US 2007/0002444 A1 describes IOLs capable of reducing chromatic aberration, and US 2004/0252274 A1 describes bifocal multiorder diffractive IOLs for vision correction.

Documents US 2019/365528 A1, WO 2009/027438 A2, AU 2017 225 592 A1, AU 2017 369 976 A1 and US 2008/300679 A1 describe different multifocal ophthalmic intraocular lenses.

In order to cover a range of conditions, human eyes adapt in at least three ways. Focus adjustment by deforming the natural eye lens is primary adaption.

When the light conditions change, the iris of the eye changes its size to adapt the pupil diameter. At the same time the spectral sensitivity of the eye changes. For example under lower ambient light levels (darker conditions), the pupil diameter is larger and the spectral sensitivity of the retina is more heavily weighted to the blue end of the visible spectrum. These adaptions, however, impair visual performance in case of eyes having an IOL.

Because of this, it is object of the invention to improve the visual performance of the patients having an IOL.

The invention is defined in claim 1. The dependent claims relate to advantageous embodiments.

An ophthalmic intraocular lens (IOL) is provided to be inserted into the human eye, e.g. as a replacement lens in cataract surgery or in case of aphakia or as an add-on lens. The ophthalmic intraocular lens comprises a lens body having a surface and at least two concentric zones around an optical axis of the ophthalmic intraocular lens, each zone having a diffractive structure. A first central zone comprises a first diffractive structure and a second ring-shaped zone comprises a second diffractive structure. The diffractive structures of the at least two zones differ from each other. In the first central zone, the surface and the first diffractive structure provide at least a first and a second focus, i.e. at least a pair of foci, for visible light at a first design wavelength. **In** the second ring-shaped zone, the surface and the second diffractive structure provide the same at least pair of foci, i.e. at least the first and second foci, however, for visible light at a second design wavelength. The first design wavelength and the second design wavelength differ from each other.

The first diffractive structure of the first central zone comprises ridges and valleys in rings around the optical axis. The second ring-shaped zone also comprises ridges and valleys in rings around the optical axis. **In** both diffractive structures, the height profile of the surface may be understood as a kind of sawtooth profile including individual heights, being peaks of the ridges. The heights are spaced by widths, being the spacing of the peaks.

The ophthalmic intraocular lens generates at least two diffraction orders for each zone. Each order generates a focal length, hence focus. Thus, a pair of foci exists for two diffraction orders of each zone. The design of the lens body, i.e. the curvature of the lens, and the design of the diffractive structures is such that for two zones the pair of foci is the same, although at different design wavelengths. For example, a first focus results from a zeroth diffraction order of the first diffractive structure and from the curvature of the surface at the first design wavelength, and a second focus results from a first diffraction order of the first diffractive structure and from the curvature of the surface at the first design wavelength. The same first focus results from a zeroth diffraction order of the second diffractive structure and from the curvature of the surface at the second design wavelength, and the same second focus results from a first diffraction order of the second diffractive structure and from the curvature of the surface at the second design wavelength.

The term "curvature" relates to the base curvature of the lens body, i.e. does not include the diffractive structure which is provided on the curved lens body. **In** the first central zone, the curvature of the surface can be different to the curvature of the surface in the second ring-shaped zone in order to obtain the same focal lengths in combination with the second diffractive structure although at different design wavelengths. The first diffractive structure differs in its widths from the second diffractive structure such that the same pair of foci is provided by the diffractive structures in both zones at different design wavelengths.

The widths of the sawtooth profile of the first and second diffractive structures and the surface of the respective ring-shaped zone are chosen to guide incoming light at the respective design wavelength to the same pairs of foci.

In embodiments, relative energy distributions between the diffraction orders resulting from the diffractive structures of the ophthalmic intraocular lens are the same in both zones. This results from the different heights in the first and in the second diffractive structure. The relative energy distributions between the two foci are identical because proportions of peaks of the relative energy distribution are identical.

In embodiments, the heights of the sawtooth profile of the first and second diffractive structures and the surface of the respective ring-shaped zone are chosen to preserve the relative energy distributions within the foci of each pair at the respective design wavelength. The relative energy distributions between the two foci are identical for visible light of one wavelength (first design wavelength) falling onto the central zone only and for visible light of another wavelength (second design wavelength) falling onto the central zone and the second ring-shaped zone within a tolerance of +/- 20 % or preferred +/- 10 % or mostly preferred +/- 5 %. Not only the absolute values of the relative energy distributions are identical within defined tolerance but also the proportions of the relative energy distribution within the pair of foci of the central and the second ring-shaped zone do not change at the different illumination conditions (within a tolerance of +/-20%, or preferred +/- 10%, or mostly preferred +/- 5%).

In embodiments, the heights get lower at increased radiuses.

Of course, each zone can generate more than a pair of foci, i.e. more than two foci. Reference to a pair of foci or two foci always includes the option to have three or even four foci.

The ophthalmic intraocular lens is adapted to the size change of the iris and the spectral sensitivity change when the light conditions change. When only the first central zone of the ophthalmic intraocular lens is illuminated, a first pupil diameter of the central zone produces the same pair of foci and relative energy distributions within the pair as a second pupil diameter does when the first central zone and the second ring-shaped zone are illuminated. Because of that, any mismatch between the relevant wavelength of sensitivity and the design wavelength of the ophthalmic intraocular lens which would result in changed focal planes and an unexpected light distribution between foci is avoided. The visual performance of the patients is not reduced when light conditions change.

The ophthalmic intraocular lens delivers two significant performance improvements over a conventional ophthalmic intraocular lens. As the relation between ambient light conditions, size of iris and spectral sensitivity is taken into account, a better performance is achieved over a wider range of illumination conditions. Additionally targeted focal distances and their relative energy distributions are both preserved when the light level changes.

Further, the ophthalmic intraocular lens is more insensitive to variations of the IOL location in the patient's eye after the implantation than conventional multifocal ophthalmic intraocular lenses are.

In an embodiment, a third concentric zone around the optical axis is interposed between the central and the second ring-shaped zone. This zone comprises a third diffractive structure composed of different heights and widths which provides in combination with the surface in the third concentric ring-shaped zone a further pair of foci for visible light at a third design wavelength. This third pair is identical to the pairs of the first and the second zones.

In a preferred embodiment, the central zone is adapted to photopic viewing conditions of the eye and/or the second ring-shaped zone is adapted to scotopic viewing conditions of the eye. By interposing a third ring-shaped zone around the optical axis between the central and the second ring-shaped zone, this third zone can be adapted for mesopic viewing conditions to maintain the produced pair of foci of the ophthalmic intraocular lens in a wider range of illumination conditions than it is achievable with a conventional multifocal ophthalmic lens.

In embodiments adjacent diffractive structures abut gap-less to each other. Then, there is either a hard or a smooth transition between the widths and heights of the sawtooth profile of adjacent diffractive structures. A "hard transition" means a direct contact between the widths and heights of the sawtooth profile of the adjacent first and second diffractive structure without any transition area between them. In case of a "smooth transition" there is a transition area between the first and second adjacent diffractive structure in which area the widths and heights of the sawtooth profile of the adjacent diffractive structures transform slowly from the one to the other structure.

In another preferred embodiment the curvature of the surface of the lens in the regions of the first and the second diffractive structure is additionally adapted to correct spherical aberration.

In another embodiment, the first and/or the second diffractive structure is adapted to correct spherical aberration with respect to the first design wavelength λ₁ and the second design wavelength λ₂.

In a preferred embodiment, the first diffractive structure in the central zone extends from the optical axis to a radius of 1.5 mm and the second diffractive structure in the second ring-shaped zone extends from the border of the central zone or from the border of any third zone to a radius of 3 mm.

In another preferred embodiment the design wavelength of the central zone is 555 nm and/or the design wavelength of the second ring-shaped zone is 507 nm.

It is understood that the features named above and those yet to be explained below can be used not only in the combinations indicated but also in other combinations or alone, without departing from the scope of the present invention.

In the following, the invention is explained in yet more detail by means of embodiment examples with reference to the attached drawings, which also disclose features essential to the invention. These embodiment examples merely serve for illustration and are not to be interpreted as limiting. For example, a description of an embodiment example with a plurality of elements or components is not to be interpreted to the effect that all of these elements or components are necessary for the implementation. Rather, other embodiment examples can also contain alternative elements and components, fewer elements or components or additional elements or components. Elements or components of different embodiment examples can be combined with each other, unless otherwise indicated. Modifications and adjustments which are described for one of the embodiment examples can also be applicable to other embodiment examples. For the avoidance of repetition, identical elements or those corresponding to each other are labelled with the same reference numbers in different figures and are not explained multiple times. The figures show:
- Fig. 1A: a sectional view of an ophthalmic intraocular lens;
- Fig. 1B: a sectional view of the ophthalmic intraocular lens in more detail with focused light beams at a first and a second wavelength;
- Fig. 2A and 2B: a top-view of the ophthalmic intraocular lens with at least two different concentric zones;
- Fig. 3: a sectional view of a surface of the ophthalmic intraocular lens with two different diffractive structures and a hard transition between;
- Fig. 4: a sectional view of the surface of the ophthalmic intraocular lens with two different diffractive structures and a smooth transition between, and
- Fig. 5: axial intensity plots for three different focal distances and two illumination conditions.

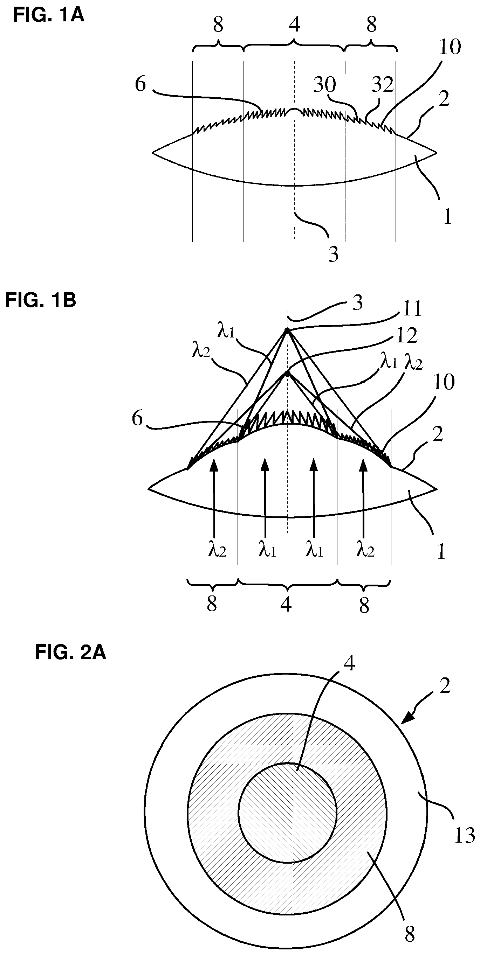

Fig. 1A shows a sectional view of an ophthalmic intraocular lens 1. Fig. 1B shows a sectional view of the ophthalmic intraocular lens in more detail with curvature of a lens body exaggerated to illustrate a focussing effect at a first design wavelength λ₁ and a second design wavelength λ₂.The ophthalmic intraocular lens comprises a lens body featuring an aspheric surface 2 of the lens body. Onto this surface 2 diffractive structures are provided in different concentric zones around an optical axis 3. A first zone which is a central zone 4 comprises a first diffractive structure 6, and a second zone which is a ring-shaped zone 8 around the central zone 4 comprises a second diffractive structure 10 (see Fig. 2A).

A top-view of the surface 2 with the two different zones 4, 8 and an outer zone 13 for mounting the lens is shown in Fig. 2A.

The two zones are adapted to different wavelengths. The central zone 4 is adapted to light of a first wavelength λ₁, whereas the outer ring-shaped zone 8 is adapted to a wavelength λ₂. Each of the zones generates at least two foci 11, 12. One of the foci 11, 12 is provided by the curvature of the surface 2, whereas the other of the two foci 11, 12 is generated by the diffractive structure provided within the respective zone 4, 8. The focus generated by the curvature can be seen as zero order diffraction of the IOL, and the focus generated by the diffractive structure can be identified as a first order of diffraction. Both zones 4, 8 generate the same pair of foci 11, 12, i.e. a first focus 11 and a second focus 12. However, the two zones 4, 8 exhibit this optical action at different design wavelengths. The design wavelength used for the central zone 4 is λ₁ and is usually longer than the design wavelength λ₂ on which the second zone 8 is based. In any case, the two design wavelengths are different. Because the two zones cover different pupil sizes (cf. Fig. 2A), the design wavelength of the first zone 4 is selected to comply with viewing conditions in which the pupil is rather small, whereas the design wavelength for the second zone 8 is selected to comply with viewing conditions at which the pupil is larger.

Of course, viewing conditions at large pupils do not only illuminate the second zone 8 but also the central zone 4. Because of that, the profile of the central zone 4 is optimized for the first design wavelength λ₁. The profile of the ring-shaped zone 8 is optimized for the second design wavelength λ₂ when the effect of the first design wavelength λ₁ is taken into account.

In other words, the design of the ring-shaped zone 8 takes into account the collective behavior of that zone and the central zone 4. Specifically, it counterbalances any shifts in focal distance and relative energy distribution resulting from the interplay of the diffractive pattern in the central zone 4 with the mismatched design wavelength λ₂ of the outer ring-shaped zone 8.

Each diffractive structure is an optical periodical structure that splits and diffracts light into several beams travelling to different directions. Looking at the height profile, each diffractive structure looks like a sawtooth profile with individual heights 32 spaced by widths 30 (cf. Figs. 1A, 3, 4). The directions of the light beams depend on the heights 32 and the surface areas resulting from the widths 30 of the sawtooth profile of the diffractive structure, and of the wavelength of the light diffracted.

The two zones 4, 8 have different optical properties. The surface 2 in the first central zone 4 is formed to focus visible light of the first design wavelength λ₁ to the first focus 11. The surface 2 in the second ring-shaped zone 8 focuses visible light at the second design wavelength λ₂ to the first focus 11. Because of that, the surface is aspheric and differs regarding curvature between the first central zone 4 and the second ring-shaped zone 8. The same first focus 11 is produced for visible light at the two different design wavelengths λ₁, λ₂ by two zones.

The diffractive structures 6, 10 have different optical properties as well. The heights and widths of the sawtooth profile of each diffractive structure 6, 10 are designed that the second focus 12 is obtained for visible light of the second design wavelength λ₂ in the second ring-shaped zone 8 and for visible light of the first design wavelength λ₁ in the first central zone 4.

Thus, the central zone 4 generates at least the first and the second focus 11, 12 in aberration corrected manner for visible light at e.g. a design wavelength of 555 nm by the curvature of the surface 2 in the central zone 4 and the first diffractive structure 6. In the second ring-shaped zone 8 the same foci 11, 12 are generated in aberration corrected manner for visible light at a different design wavelength, e.g. 507 nm, by the curvature of the surface 2 in the second ring-shaped zone 8 and the second diffractive structure 10.

Hence, the central zone 4 is optimized for photopic viewing conditions of the eye and the second ring-shaped zone 8 is optimized for scotopic viewing conditions of the eye. Photopic vision of the eye is vision of the eye under well-lit conditions, normally usual daylight light at high intensity. The second ring-shaped zone 8 is optimized for scotopic viewing conditions of the eye. Scotopic vision is vision of the eye under low light conditions.

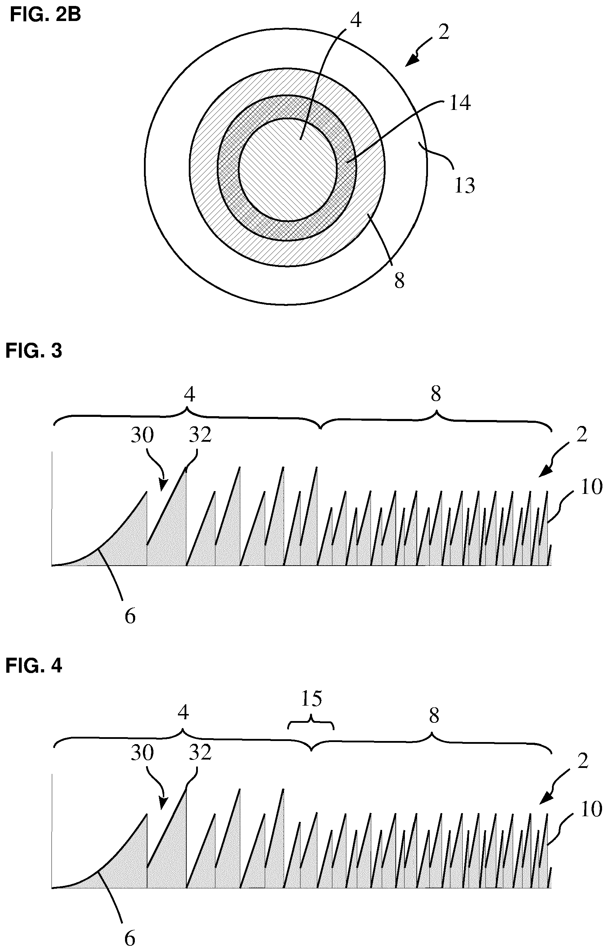

Fig. 2B relates to an embodiment comprising a third concentric ring-shaped zone 14, which is interposed between the central zone 4 and the second ring-shaped zone 8. This third ring-shaped zone 14 comprises a third diffractive structure. This third diffractive structure provides equally the at least first and second foci 11, 12, however for visible light at a third design wavelength. The third ring-shaped zone 14 is optimized for mesopic viewing conditions. Mesopic vision is the type of vision that takes place in intermediate lighting conditions.

Fig. 3 shows a sectional view of the diffractive structures 6, 10 on the surface 2 of the ophthalmic intraocular lens 1 shown in Fig. 1A and B and 2A. The diagram shows the height profiles of the first and the second diffractive structure 6 and 10. The curvature of the surface 2 is not shown to keep Fig. 3 simple. The diagram starts at the optical axis 3 in the centre of the pupil and shows the diffractive structures up to a radius of 3 mm. The heights of the diffractive structures are a few microns.

The first diffractive structure 6 is designed for photopic spectral sensitivity and provided in inner pupil regions, namely in the central zone 4. The second diffractive structure 10 is designed for scotopic spectral sensitivity and is provided in outer pupil regions, namely in the second ring-shaped zone 8. The second diffractive structure 10 comprises lower heights and smaller widths of the sawtooth profile than the first diffractive structure 6 to produce the first and second foci 11, 12 at shorter wavelengths which are relevant for scotopic viewing conditions.

In the embodiment of Fig. 3, the diffractive structures 6, 10 adjoin to each other gap-less by a hard transition. In another case, shown in Fig. 4, a smooth transition 15 is provided between the first diffractive structure 6 and the second diffractive structure 10 to avoid the hard transition between the widths and heights of the sawtooth profile of the first and the second diffractive structure 6 and 10. Both transitions are options for the three zone embodiment of Fig. 2B.

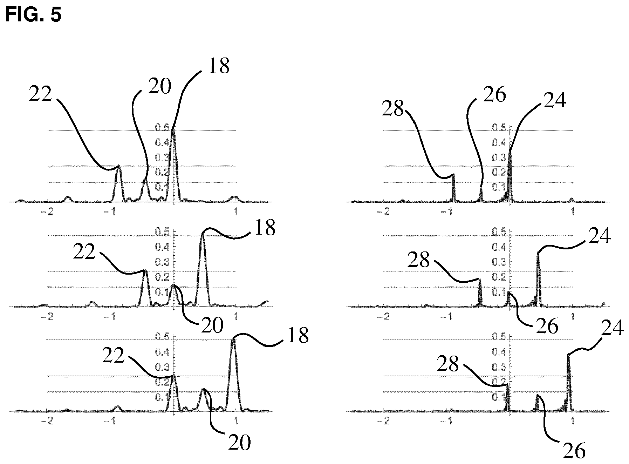

Fig. 5 shows plots of monochromatic axial intensity for three different viewing distances at two illumination conditions. Each column shows the axial intensity at the retina found for an object at infinity, at 800 mm and at 400 mm in the top, central and bottom line, respectively. The horizontal coordinate is shown in mm units and its origin is chosen to sit at the retina.

On the left side of Fig. 5, the three graphs correspond to the viewing conditions in a wavelength of 555 nm and an iris diameter of 3 mm. In this case, a first peak 18, a second peak 20 and a third peak 22 correspond to action of the curved surface 2 and the first diffractive structure 6. On the right side of Fig. 5, the three graphs correspond to the viewing conditions at a wavelength of 507 nm and an iris diameter of 6 mm. In this case, a first peak 24, a second peak 26 and a third peak 28 correspond to action of the curved surface 2, the first structure 6 and the second diffractive structure 10.

The first peak 18 and 24 corresponds to light of the zero order reaching the retina. Because of that, watching an object at infinity (top line of Fig. 5) results in the first peak 18 and 24 exactly onto the retina (y-axis). The second peak 20 and 26 corresponds to light of the first order reaching the retina. Because of that, watching an object at 800 **mm** distance (central line of Fig. 5) results in the second peak 20 and 26 exactly onto the retina (y-axis). The third peak 22 and 28 corresponds to light of the second order reaching the retina. Because of that, watching an object at 400 **mm** distance (bottom line of Fig. 5) results in the third peak 22 and 28 exactly onto the retina (y-axis).

The widths and the heights of the sawtooth profile of the diffractive structure are modified to keep at least two foci unchanged and furthermore the relative energy distributions within the at least two foci constant even when the light conditions are changing.

## Claims

1. An ophthalmic intraocular lens to be inserted into the human eye, the intraocular lens (1) comprising:
a lens body having a surface (2),
at least two concentric zones on the surface (2), with
a first central zone (4) having a first diffractive structure (6) comprising widths (30) and heights (32), and
a second ring-shaped zone (8) having a second diffractive structure (10) comprising widths (30) and heights (32),
**characterized in that**
a curvature of the surface (2) in the first central zone (4) and the first diffractive structure (6) are configured to provide at least a first and a second focus (11, 12) for visible light at a first design wavelength (λ₁),
a curvature of the surface (2) in the second ring-shaped zone (8) and the second diffractive structure (10) are configured to provide the same at least first and second focus (11, 12) for visible light at a second design wavelength (λ₂), and
the first design wavelength (λ₁) and the second design wavelength (λ₂) are different.

2. The ophthalmic intraocular lens according to claim 1, **characterized in that** the first central zone (4) is configured to photopic viewing conditions of the eye.

3. The ophthalmic intraocular lens according to claim 1 or 2, **characterized in that** the second ring-shaped zone (8) is configured to scotopic viewing conditions of the eye.

4. The ophthalmic intraocular lens according to any of the above claims, **characterized in that** a third concentric ring-shaped zone (14) having a third diffractive structure comprising widths and heights is added, the surface (2) and the third diffractive structure is configured to provide the at least first and second foci (11, 12) for visible light at a third design wavelength.

5. The ophthalmic intraocular lens according to claim 4, **characterized in that** the third concentric ring-shaped zone (14) is configured to mesopic viewing conditions.

6. The ophthalmic intraocular lens according to any of the above claims, **characterized in that** adjacent ones of the diffractive structures (6, 10) abut gap-less to each other.

7. The ophthalmic intraocular lens according to claim 6, **characterized in that** the widths (30) and heights (32) of adjacent ones of the diffractive structures (6, 10) are in direct contact without any transition area (15) between them.

8. The ophthalmic intraocular lens according to claim 6, **characterized in that** the widths (30) and heights (32) of the first diffractive structure (6) and the widths (30) and heights (32) of adjacent ones of the diffractive structures (6, 10) are connected by a transition area (15) between them.

9. The ophthalmic intraocular lens according to any of the above claims, **characterized in that** the curvature of the surface (2) in the first (4) and the second zone (8) is configured to correct spherical aberration.

10. The ophthalmic intraocular lens according to any of the above claims, **characterized in that** the first and/or the second diffractive structure (6, 10) is configured to a correction of spherical aberration.

11. The ophthalmic intraocular lens according to any of the above claims, **characterized in that** the first diffractive structure (6) extends from the optical axis (3) to a radius of 1.5 mm to define the central zone (4) and the second diffractive structure (10) extends from a border of the central zone (4) or from a border of any third zone to a radius of 3 mm to define the second ring-shaped zone (8).

12. The ophthalmic intraocular lens according to any of the above claims, **characterized in that** the design wavelength for the central zone (4) is 555 nm and the design wavelength of the second ring-shaped zone (8) is 507 nm.

13. The ophthalmic intraocular lens according to any of the above claims, **characterized in that** the relative energy distribution between the two foci is identical for visible light of the first design wavelength (λ₁) falling onto the central zone (4) only and for visible light of the second design wavelength (λ₂) falling onto the central zone (4) and the second ring-shaped zone (8), in each case within a tolerance of +/- 20% or preferred +/- 10% or mostly preferred +/- 5%.

## Patentansprüche

1. Ophthalmische Intraokularlinse für das menschliche Auge, wobei die Intraokularlinse (1) umfasst:
- einen Linsenkörper mit einer Oberfläche (2),
- mindestens zwei konzentrische Zonen auf der Oberfläche (2), wobei
- eine erste, zentrale Zone (4) eine erste diffraktive Struktur (6) aufweist, die Weiten (30) und Erhebungen (32) aufweist, und
- eine zweite, ringförmige Zone (8) eine zweite diffraktive Struktur (10) aufweist, die Weiten (30) und Erhebungen (32) aufweist,
**dadurch gekennzeichnet, dass**
- eine Krümmung der Oberfläche (2) in der ersten, zentralen Zone (4) und die erste, diffraktive Struktur (6) so konfiguriert sind, dass sie mindestens einen ersten und einen zweiten Fokus (11, 12) für sichtbares Licht bei einer ersten Auslegungswellenlänge (λ₁) bereitstellen,
- eine Krümmung der Oberfläche (2) in der zweiten ringförmigen Zone (8) und die zweite diffraktive Struktur (10) so konfiguriert sind, dass sie dieselben mindestens ersten und zweiten Foki (11, 12) für sichtbares Licht bei einer zweiten Auslegungswellenlänge (λ₂) bereitstellen, und
- die erste Auslegungswellenlänge (λ₁) und die zweite Auslegungswellenlänge (λ₂) unterschiedlich sind.

2. Ophthalmische Intraokularlinse nach Anspruch 1, **dadurch gekennzeichnet, dass** die erste zentrale Zone (4) auf photopische Sehbedingungen des Auges ausgelegt ist.

3. Ophthalmische Intraokularlinse nach Anspruch 1 oder 2, **dadurch gekennzeichnet, dass** die zweite, ringförmige Zone (8) auf skotopische Sehbedingungen des Auges ausgelegt ist.

4. Ophthalmische Intraokularlinse nach einem der vorstehenden Ansprüche, **dadurch gekennzeichnet, dass** eine dritte, konzentrische ringförmige Zone (14) mit einer dritten diffraktiven Struktur, die Weiten und Erhebungen umfasst, hinzugefügt ist, wobei die Oberfläche (2) und die dritte diffraktive Struktur so ausgelegt sind, dass sie den zumindest ersten und zweiten Fokus (11, 12) für sichtbares Licht bei einer dritten Auslegungswellenlänge bereitstellen.

5. Ophthalmische Intraokularlinse nach Anspruch 4, **dadurch gekennzeichnet, dass** die dritte, konzentrische ringförmige Zone (14) auf mesopische Sehbedingungen ausgelegt ist.

6. Ophthalmische Intraokularlinse nach einem der vorstehenden Ansprüche, **dadurch gekennzeichnet, dass** benachbarte der diffraktiven Strukturen (6, 10) lückenlos aneinander angrenzen.

7. Ophthalmische Intraokularlinse nach Anspruch 6, **dadurch gekennzeichnet, dass** die Weiten (30) und Erhebungen (32) benachbarter diffraktiver Strukturen (6, 10) in direktem Kontakt stehen, ohne dass zwischen ihnen ein Übergangsbereich (15) vorhanden ist.

8. Ophthalmische Intraokularlinse nach Anspruch 6, **dadurch gekennzeichnet, dass** die Weiten (30) und Erhebungen (32) der ersten, diffraktiven Struktur (6) und die Weiten (30) und Erhebungen (32) benachbarter diffraktiver Strukturen (6, 10) durch einen Übergangsbereich (15) zwischen ihnen verbunden sind.

9. Ophthalmische Intraokularlinse nach einem der vorstehenden Ansprüche, **dadurch gekennzeichnet, dass** die Krümmung der Oberfläche (2) in der ersten (4) und der zweiten Zone (8) zur Korrektur der sphärischen Aberration ausgebildet ist.

10. Ophthalmische Intraokularlinse nach einem der vorstehenden Ansprüche, **dadurch gekennzeichnet, dass** die erste und/oder die zweite diffraktive Struktur (6, 10) zur Korrektur der sphärischen Aberration ausgebildet ist.

11. Ophthalmische Intraokularlinse nach einem der vorstehenden Ansprüche, **dadurch gekennzeichnet, dass** sich die erste diffraktive Struktur (6) von der optischen Achse (3) bis zu einem Radius von 1,5 mm erstreckt, um die zentrale Zone (4) zu definieren, und sich die zweite diffraktive Struktur (10) von einem Rand der zentralen Zone (4) oder von einem Rand einer beliebigen dritten Zone bis zu einem Radius von 3 mm erstreckt, um die zweite, ringförmige Zone (8) zu definieren.

12. Ophthalmische Intraokularlinse nach einem der vorstehenden Ansprüche, **dadurch gekennzeichnet, dass** die Auslegungswellenlänge für die zentrale Zone (4) 555 nm und die Auslegungswellenlänge für die zweite ringförmige Zone (8) 507 nm beträgt.

13. Ophthalmische Intraokularlinse nach einem der vorstehenden Ansprüche, **dadurch gekennzeichnet, dass** die relative Energieverteilung zwischen den beiden Foki für sichtbares Licht der ersten Auslegungswellenlänge (λ₁), das nur auf die zentrale Zone (4) fällt, und für sichtbares Licht der zweiten Auslegungswellenlänge (λ₂), das auf die zentrale Zone (4) und die zweite, ringförmige Zone (8) fällt, jeweils innerhalb einer Toleranz von +/- 20 % oder vorzugsweise +/- 10 % oder am meisten bevorzugt +/- 5 % identisch ist.

## Revendications

1. Lentille intraoculaire ophtalmique destinée à l'œil humain, ladite lentille intraoculaire (1) comprenant :
- un corps de lentille présentant une surface (2),
- au moins deux zones concentriques sur la surface (2), dans lesquelles
- une première zone centrale (4) comporte une première structure diffractive (6) présentant des creux (30) et des bosses (32), et
- une deuxième zone annulaire (8) comporte une deuxième structure diffractive (10) qui présente des creux (30) et des bosses (32),
**caractérisée en ce que**
- une courbure de la surface (2) dans la première zone centrale (4) et la première structure diffractive (6) sont configurées de manière à fournir au moins un premier et un deuxième foyers (11, 12) pour la lumière visible à une première longueur d'onde de conception (λ₁),
- une courbure de la surface (2) dans la deuxième zone annulaire (8) et la deuxième structure diffractive (10) sont configurées de manière à fournir les mêmes au moins premier et deuxième foyers (11, 12) pour la lumière visible à une deuxième longueur d'onde de conception (λ₂), et
- la première longueur d'onde de conception (λ₁) et la deuxième longueur d'onde de conception (λ₂) sont différentes.

2. Lentille intraoculaire ophtalmique selon la revendication 1, **caractérisée en ce que** la première zone centrale (4) est conçue pour les conditions de vision photopique de l'œil.

3. Lentille intraoculaire ophtalmique selon la revendication 1 ou 2, **caractérisée en ce que** la deuxième zone annulaire (8) est conçue pour les conditions de vision scotopique de l'œil.

4. Lentille intraoculaire ophtalmique selon l'une des revendications précédentes, **caractérisée en ce qu'**une troisième zone annulaire concentrique (14) comportant une troisième structure diffractive comprenant des creux et des bosses est ajoutée, la surface (2) et la troisième structure diffractive étant conçues pour fournir au moins les premier et deuxième foyers (11, 12) pour la lumière visible à une troisième longueur d'onde de conception.

5. Lentille intraoculaire ophtalmique selon la revendication 4, **caractérisée en ce que** la troisième zone annulaire concentrique (14) est conçue pour des conditions de vision mésopique.

6. Lentille intraoculaire ophtalmique selon l'une des revendications précédentes, **caractérisée en ce que** les structures diffractives (6, 10) adjacentes sont contiguës sans intervalle.

7. Lentille intraoculaire ophtalmique selon la revendication 6, **caractérisée en ce que** les creux (30) et les reliefs (32) de structures diffractives adjacentes (6, 10) sont en contact direct, sans qu'il y ait de zone de transition (15) entre eux.

8. Lentille intraoculaire ophtalmique selon la revendication 6, **caractérisée en ce que** les creux (30) et les reliefs (32) de la première structure diffractive (6) et les creux (30) et les reliefs (32) de structures diffractives adjacentes (6, 10) sont reliés entre eux par une zone de transition (15).

9. Lentille intraoculaire ophtalmique selon l'une des revendications précédentes, **caractérisée en ce que** la courbure de la surface (2) dans la première (4) et la deuxième zone (8) est conçue pour corriger l'aberration sphérique.

10. Lentille intraoculaire ophtalmique selon l'une des revendications précédentes, **caractérisée en ce que** la première et/ou la deuxième structure diffractive (6, 10) est conçue pour corriger l'aberration sphérique.

11. Lentille intraoculaire ophtalmique selon l'une des revendications précédentes, **caractérisée en ce que** la première structure diffractive (6) s'étend de l'axe optique (3) jusqu'à un rayon de 1,5 mm pour définir la zone centrale (4), et la deuxième structure diffractive (10) s'étend d'un bord de la zone centrale (4) ou d'un bord d'une troisième zone quelconque jusqu'à un rayon de 3 mm pour définir la deuxième zone annulaire (8).

12. Lentille intraoculaire ophtalmique selon l'une des revendications précédentes, **caractérisée en ce que** la longueur d'onde de conception pour la zone centrale (4) est de 555 nm et la longueur d'onde de conception pour la deuxième zone annulaire (8) est de 507 nm.

13. Lentille intraoculaire ophtalmique selon l'une des revendications précédentes, **caractérisée en ce que** la répartition relative de l'énergie entre les deux foyers pour la lumière visible de la première longueur d'onde de conception (λ₁), qui tombe uniquement sur la zone centrale (4), et pour la lumière visible de la deuxième longueur d'onde de conception (λ₂), qui tombe sur la zone centrale (4) et sur la deuxième zone annulaire (8) est identique, dans une tolérance de +/- 20 %, ou de préférence de +/- 10 %, ou de manière encore plus préférée de +/- 5 %.
